# EUROPEAN PATENT SPECIFICATION

(11) **EP 2 964 183 B1**
(45) Date of publication and mention of the grant of the patent: **30.08.2017**
(21) Application number: 14706846.4
(22) Date of filing: 27.02.2014
(51) Int. Cl.: A61K 8/362, A61Q 5/04

(54) **METHOD OF TREATING HAIR**
VERFAHREN ZUR HAARBEHANDLUNG
PROCÉDÉ DE TRAITEMENT DES CHEVEUX

(30) Priority: 04.03.2013 EP 13157663; 25.04.2013 EP 13165323
(43) Date of publication of application: 13.01.2016
(73) Proprietor: Unilever PLC, London, Greater London EC4Y 0DY (GB); Unilever N.V., 3013 AL Rotterdam (NL)
(72) Inventor: PAUL, Prem, Kumar, Cheyalazhagan, Wirral Merseyside CH63 3JW (GB)
(74) Representative: James, Helen Sarah
(86) International application number: PCT/EP2014/053811
(87) International publication number: WO 2014/135433

(56) References cited:
- WO-A1-2005/025524
- WO-A1-2013/174575
- WO-A2-2010/049434
- US-A- 3 025 218
- US-A- 4 409 204

## Description

The invention relates to a hair method of hair straightening.

Permanent hair straightening compositions that are on the market are based on chemical treatment of the hair in a two-step process using thiol- or hydroxide-based reducing agents followed by a neutralisation or oxidation step. Such systems have various negatives associated with them; in that the process itself is difficult to conduct, in many instances this straightening process is undertaken by a qualified hairdresser in a professional salon. Furthermore the straightening process damages the hair, has an unpleasant odour and can cause irritation to the scalp.

WO2010049434 discloses a process for straightening or relaxing hair using a composition having a pH of from 8 to 11.5 and comprising at least one weak acid chosen from monocarboxylic, dicarboxylic and tricarboxylic acids. Preferred are dicarboxylic acids such as malic and tartaric acids. The only tricarboxylic acids mentioned are not unsaturated.

WO2005025524 discloses a hair straightening composition comprising reducing agents and alpha-hydroxy acids. Particularly preferred alpha-hydroxy acids are citric and tartaric acid.

US3025218 discloses the use of a non-toxic organic acid in waving hair, in waving solution comprising a mercaptan reducing agent and excess ammonium hydroxide. Aconitic acid is mentioned as one of the acids.

US4409204 discloses a method for the after-treatment of permanently deformed hair which uses a mixture of glyoxylic acid and an activated unsaturated acid. Aconitic acid is given as an example of the activated acid. The present invention has now found that hair can be straightened in a way that mitigates damage associated with the above reducing based systems.

### Summary of Invention

The present invention relates to a method of straightening hair comprising the step of applying to the hair a composition comprising from 0.5 to 20 wt% of an unsaturated tricarboxylic acid having a carbon chain length from C2 to C8.

### Description of Invention

Preferably there should be no more than 3 carbon atoms in between at least two of the carboxylic acid groups; most preferably the unsaturated tricarboxylic acid is aconitic acid. Aconitic acid may be in its *trans* or *cis* form; however trans is preferred.

Preferably the level of tricarboxylic acid is greater than 1 wt% of the total composition; more preferably greater than 2 wt%; most preferably 4 wt% or greater.

Preferably the composition of the invention comprises less than 0.1 wt% of a reducing agent; more preferably less than 0.1 wt% of a thiol reducing agent.

It is preferred if the compositions of the invention have a pH 3 or below at 20°C; more preferably pH 2.5 or below at 20°C.

Hair care compositions of the present invention can comprise a carrier, or a mixture of such carriers, which are suitable for application to the hair. The carriers are present at from about 0.5% to about 99.5%, preferably from about 5.0% to about 99.5%, more preferably from about 10.0% to about 98.0%, of the composition. As used herein, the phrase "suitable for application to hair" means that the carrier does not damage or negatively affect the aesthetics of hair or cause irritation to the underlying skin.

Compositions according to the invention are preferably aqueous compositions, in some instances intended to be applied to the hair after shampooing and rinsing. Preferably the compositions are massaged into dry hair, left on the hair for at least 5 minutes (the hair may be heated) followed by further rinsing with water prior and combing. By aqueous composition, it is meant that the compositions of the invention comprise 60% by weight or more of water, preferably 70% or more, more preferably 80% or more.

When the hair care composition is a lotion, cream, tonic, gel, or mousse the preferred solvents include water, ethanol, volatile silicone derivatives, and mixtures thereof. The solvents used in such mixtures may be miscible or immiscible with each other. Lotions, creams, and gels are preferred.

Mousses can also utilise any of the conventional propellants to deliver the material as a foam (in the case of a mousse). Examples of suitable propellants include materials such as trichlorofluoromethane, dichlorodifluoromethane, difluoroethane, dimethylether, propane, n-butane or isobutane. A product having a low viscosity may also utilise an emulsifying agent. Examples of suitable emulsifying agents include nonionic, cationic, anionic surfactants, or mixtures thereof. If such an emulsifying agent is used, it is preferably present at a level of from about 0.01% to about 7.5% by weight based on total weight of the composition. The level of propellant can be adjusted as desired but is generally from about 3% to about 30% by weight based on total weight for mousse.

Hair styling creams or gels also typically contain a structurant or thickener, typically in an amount of from 0.01% to 10% by weight.

Compositions according to the invention comprise a buffer or pH adjuster. Preferred buffers or pH adjusters include weak acids and bases such glycine/sodium hydroxide, lactic acid, succinic acid, acetic salt and salts thereof. Frequently a mixture of buffering systems is used.

The formulation may include conditioning materials such as surfactants, cationic conditioners suitable for hair, quaternary silicone polymers, silicone based conditioners and their emulsions, and amino functional silicones and their emulsions.

Further general ingredients suitable for all product forms include, sun-screening agents, anti-dandruff actives, carboxylic acid polymer thickeners and emulsifiers for emulsifying the various carrier components of the compositions of the invention.

In some aspects of this invention it is highly desirable if the composition comprises a styling aid.

Particularly useful as styling aids with this invention are hair styling polymers. Hair styling polymers are well known articles of commerce and many such polymers are available commercially which contain moieties which render the polymers cationic, anionic, amphoteric or nonionic in nature. The polymers may be synthetic or naturally derived.

The amount of the hair styling polymer may range from 0.1 to 10%, preferably 0.5 to 8 %, more preferably 0.75 to 6% by weight based on total weight of the composition.

The compositions of the present invention may also contain adjuncts suitable for hair care. Generally such ingredients are included individually at a level of up to 2, preferably up to 1 wt% of the total composition. Suitable hair care adjuncts, include amino acids, sugars and ceramides.

Although the product may be in any form suitable for application to the hair it is preferable if it is a rinse off product. Products used to condition the hair are especially preferred.

In use the composition of the invention is preferably applied to the hair and left on the hair for at least, 5 minutes, preferably at least 10 minutes, more preferably at least 15 minutes. Preferably the product is rinsed off 90 minutes after application, more preferably 60 minutes after application, most preferably this product is rinsed off 40 minutes after application.

The following non-limiting Examples further illustrate the preferred embodiments of the invention. All percentages referred to in the examples and throughout this specification are by weight based on total weight unless otherwise indicated.

### Example 1

Dark brown European wavy#6 switches of length 25 cm and weight 2gms, were dosed with 2ml each of different levels of trans aconitic acid solutions. They were combed straight and left to develop for at least 20 minutes. They were subsequently rinsed for 30 seconds under the tap. They were then combed straight and left to dry overnight. When dry the switches were combed straight and pictures taken. All switches looked straight without the waviness of the pretreated switches. The volume of the switches shows the straightening benefit of trans aconitic acid at different levels. (Here volume refers to the projection of the switch image on to the screen and is given in mm²).

| **Treatment** | **Volume in mm^2** | **%benefit over water** |
|---|---|---|
| water | 14278 | 0 |
| 5% Citric acid at pH 2 | 9481 | 33.6 |
| 5% trans Aconitic acid at pH 2 | 8456 | 40.8 |
| 2.5% trans Aconitic acid at pH 2 | 10763 | 24.6 |
| 1% trans Aconitic acid at pH 2 | 11363 | 20.4 |
| 0.5% trans Aconitic acid at pH 2 | 12073 | 15.4 |
| 5% trans Aconitic acid at pH 3 | 11812 | 17.3 |

From the table it can be seen that trans aconitic acid gives hair straightening benefits when applied to hair.

## Claims

1. A method of straightening hair comprising the step of applying to the hair a composition comprising from 0.5 to 20 wt% of an unsaturated tricarboxylic acid having a carbon chain length from C2 to C8.

2. A method according to claim 1 in which the unsaturated tricarboxylic acid is aconitic acid.

3. A method according to any preceding claim in which the level of tricarboxylic acid is greater than 4 wt% of the total composition.

4. A method according to any preceding claim in which the pH of the composition is pH 3 or below at 20°C.

5. A method according to any preceding claim in which the pH of the composition is pH 2.5 or below at 20°C.

6. A method according to any preceding claim in which the composition comprises less than 0.1 wt% of a reducing agent.

7. A method according to claim 6 in which the product is left on the hair for 5 to 30 minutes and then rinsed from the hair.

## Patentansprüche

1. Verfahren zur Haarglättung, umfassend den Schritt des Auftragens einer Zusammensetzung auf das Haar, die 0,5 bis 20 Gew.-% einer ungesättigten Tricarbonsäure einer Kohlenstoffkettenlänge von C2 bis C8 umfasst.

2. Verfahren nach Anspruch 1, in welchem die ungesättigte Tricarbonsäure Aconitsäure darstellt.

3. Verfahren nach irgendeinem vorhergehenden Anspruch, in welchem die Konzentration der Tricarbonsäure größer als 4 Gew.-% der Gesamtzusammensetzung ist.

4. Verfahren nach irgendeinem vorhergehenden Anspruch, in welchem der pH der Zusammensetzung bei 20°C 3 oder weniger ist.

5. Verfahren nach irgendeinem vorhergehenden Anspruch, in welchem der pH der Zusammensetzung bei 20°C 2,5 oder weniger ist.

6. Verfahren nach irgendeinem vorhergehenden Anspruch, in welchem die Zusammensetzung weniger als 0,1 Gew.-% eines Reduktionsmittels umfasst.

7. Verfahren nach Anspruch 6, in welchem das Produkt 5 bis 30 Minuten lang auf dem Haar belassen und dann von dem Haar ausgespült wird.

## Revendications

1. Procédé de raidissement des cheveux comprenant l'étape d'application aux cheveux d'une composition comprenant de 0,5 à 20 % en masse d'un acide tricarboxylique insaturé ayant une longueur de chaîne carbonée de C2 à C8.

2. Procédé selon la revendication 1, dans lequel l'acide tricarboxylique insaturé est l'acide aconitique.

3. Procédé selon l'une quelconque des revendications précédentes, dans lequel la teneur d'acide tricarboxylique est supérieure à 4 % en masse de la composition totale.

4. Procédé selon l'une quelconque des revendications précédentes, dans lequel le pH de la composition est pH 3 ou inférieur à 20°C.

5. Procédé selon l'une quelconque des revendications précédentes, dans lequel le pH de la composition est pH 2,5 ou inférieur à 20°C.

6. Procédé selon l'une quelconque des revendications précédentes, dans lequel la composition comprend moins de 0,1 % en masse d'un agent réducteur.

7. Procédé selon la revendication 6, dans lequel le produit est laissé sur les cheveux pendant de 5 à 30 minutes et est ensuite rinçé des cheveux.
